# EUROPEAN PATENT SPECIFICATION

(11) **EP 1 776 354 B1**
(45) Date of publication and mention of the grant of the patent: **21.04.2010**
(21) Application number: 04770686.6
(22) Date of filing: 11.08.2004
(51) Int. Cl.: C07D 311/58

(54) **A NOVEL PROCESS FOR PREPARATION OF NEBIVOLOL INTERMEDIATES**
NEUES VERFAHREN ZUR HERSTELLUNG VON NEBIVOLOLZWISCHENPRODUKTEN
PROCESSUS NOVATEUR DE PRÉPARATION D'INTERMÉDIAIRES DU NÉBIVOLOL

(43) Date of publication of application: 25.04.2007
(73) Proprietor: Hetero Drugs Limited, Hyderabad, Andhrapradesh. Hyderabad 500 018 (IN)
(72) Inventor: PARTHASARADHI REDDY, bandi Hetero House, Hyderabad 5 00018 (IN); RATHNAKAR REDDY, kura Hetero Drugs Limited (R & D), Hyderabad 500018 (IN); RAJI REDDY, rapolu Hetero Drugs Limited (R & D), Hyderabad 500018 (IN); MURALIDHARA REDDY, dasari Hetero Drugs Limited, Hyderabad 500018 (IN); SRINIVAS REDDY, itiyala Hetero House, Hyderabad 500018 (IN)
(74) Representative: Thomas, Simon
(86) International application number: PCT/IN2004/000241
(87) International publication number: WO 2006/016376

(56) References cited:
- EP-A1- 0 334 429
- EP-A2- 0 145 067

## Description

### FIELD OF THE INVENTION

The present invention relates to a process for separation of desired diastereomeric pair from a mixture of diastereomeric pairs thereby obtaining nebivolol intermediates.

### BACKGROUND OF THE INVENTION

EP Patent No. 0145067 disclosed 2,2'-iminobisethanol derivatives. The compounds are antihypertensive agents. Among them nebivolol, chemically (+)-[2R*[1S*,5S*(S*)]]-α,α'-[iminobis(methylene)]bis[6-fluoro-3,4-dihydro-2H-1-benzopyran-2-methanol]_is the most important antihypertensive agent. Nebivolol is represented by the following structure:

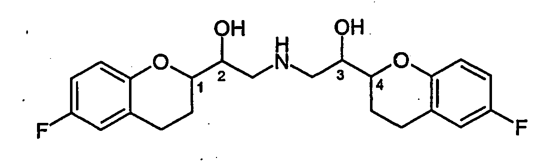

The above structure has four stereogenic centers, which are indicated with No. 1, 2, 3 and 4. Nebivolol is a mixture of equal amounts of 2 enantiomers having respectively the SRRR- and the RSSS-configuration.

Processes for preparations of nebivolol and related compounds were described in EP Patent No. 0145067 and EP Patent No. 0334429. According to the processes described in these patents, chromatographic separations are required for the separation of diastereomeric pairs at the intermediate stage or at the final stage. The chromatographic separations involve additional operations, additional expensive setup adding to the cost of production. US Patent No. 5,759, 580 described the separation of (±)-[2R*[1S*, 5S*(S*)]]-α,α'-[Iminobis(methylene)]bis[6-fluoro-3,4-dihydro-2H-1-benzopyran-2-methanol] hydrochloride (nebivolol hydrochloride) from the mixture of (±)-[2R*[1S*, 5S*(S*)]]+[2R*[1S*,5R*(R*)]]-α,α'-[iminobis(methylene)]bis[6-fluoro-3,4-dihydro-2H-1-benzopyran-2-methanol]. The yield of the nebivolol hydrochloride is extremely low (6.6%).

We have discovered that when N-protected compound of formula: 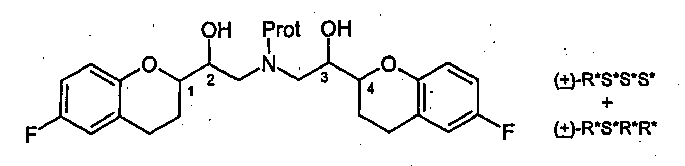 wherein -Prot is a protecting group, is converted into a salt of it, the salts can be subjected to fractional crystallization of the desired diastereomeric pair from the mixture of diastereomeric pairs. The separation of the diastereomers of these N-protected compounds by crystallization is not disclosed in the prior art. The separated diastereomeric pair is a useful intermediate for the preparation of nebivolol.

Fractional crystallization also allows the purification of the N-protected compounds from the reaction mass, thereby avoiding multiple purifications of crude nebivolol.

### DETAILED DESCRIPTION OF THE INVENTION

The present invention provides a novel process for preparing acid additional salts of compounds of formula I: 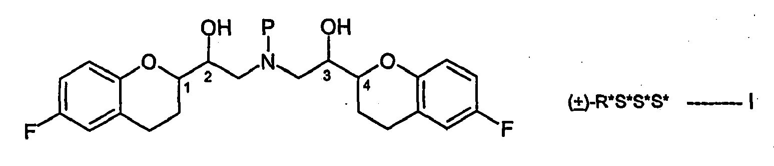 wherein .

P is -allyl or 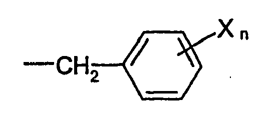 wherein
X each independently is halo, nitro or C₁-C₃ alkyl and n is 0 - 5; which comprises:
a) treating a mixture containing racemic diastereomers of a compound of formula II: 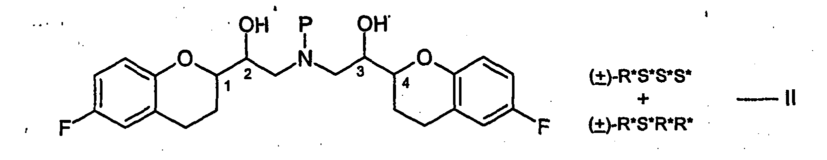 wherein P is as defined in formula I;
   with a suitable acid to form the corresponding acid addition salt;
b) subjecting the acid addition salt obtained in step (a) to the fractional crystallization from an alcoholic solvent, ketonic solvent, acetonitrile, dimethylformamide, dimethylsulfoxide and tetrahydrofuran or a mixture thereof to obtain the diastereomeric pair of compounds of formula I.

Stereochemical description describing the configurations at chiral centers used here is in the order (1,2,3 and 4) mentioned in the structure. Thus, for example, the stereochemical description R*S*S*S* sown in the formula I refers to R* configurations at the carbon '1', S* configuration at 2 and so on and R*S*S*S* has the meaning shown below.

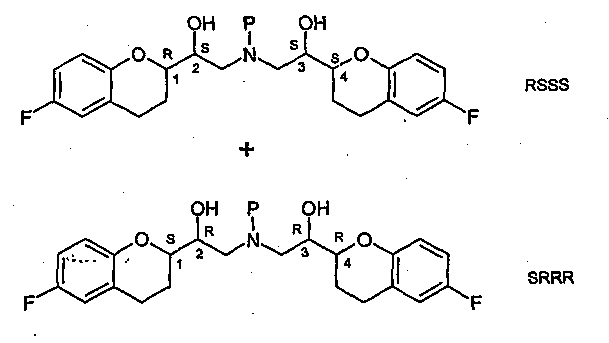

Alcoholic solvents are selected from the group consisting of C₁ to C₅ - alcohols. Preferable alcoholic solvents are methanol, ethanol, propanol and isopropyl alcohol.

Ketonic solvents are selected from the group C₃ to C₈ -ketones. Preferable ketonic solvents are acetone, methyl isobutyl ketone and methyl tert-butyl ketone.

The acid addition salts are prepared by treating the mixture containing compounds of formula II with the corresponding acids in a solvent by conventional means. The suitable acids are inorganic acids, for example, hydrogen halides, nitric acid, phosphoric acid; and organic acids such as carboxylic acids, sulfonic acids. The examples for carboxylic acids that can be mentioned are acetic acid, propanoic acid, formic acid, hydroxyacetic acid, 2-hydroxy propanoic acid, 2-oxbpropanoic acid, propanedioic acid, butanedioic acid, (Z)-2-butenedioic acid, (E)-2-butenedioic acid, 2-hydroxy butanedioic acid. The examples for sulfonic acids that can be mentioned are methane sulfonic acid, toluene sulfonic acid and benzene sulfonic acid.

The step (a) is preferably carried out in an organic solvent. The selection of the solvent is not critical. The solvents may be selected from the group consisting of C₁ to C₅ -alcohols, C₃ to C₈ -ketones, C₂ to C₈ -esters, acetonitrile, tetrahydrofuran, dimethylformamide, dimethylsulfoxide, dioxane, aromatic hydrocarbons, C₁ to C₅ -halogenated hydrocarbons and C₂ to C₈ -ethers and a mixture thereof. Preferable alcoholic solvents are methanol, ethanol, propanol and isopropyl alcohol; preferable ketonic solvents are acetone, methyl ethyl ketone, methyl isobutyl ketone, methyl tert-butyl ketone and diethyl ketone; preferable ester solvents are ethyl acetate, methyl acetate, isopropyl acetate, tert-butyl methyl acetate and ethyl formate; preferable aromatic hydrocarbon solvents are benzene, toluene and xylene; preferable halogenated hydrocarbon solvents are methylene chloride, chloroform, carbontetrachloride and ethylene dichloride; and preferable ether solvents are tert-butyl methyl ether and diethyl ether. Most preferable solvents are methanol, ethanol, propanol, isopropyl alcohol, acetone, methyl ethyl ketone, methyl isobutyl ketone, methyl tert-butyl ketone, diethyl ketone, acetonitrile, dimethylformamide, dimethylsulfoxide and tetrahydrofuran.

Preferably; fractional crystallization may be carried out in essentially anhydrous conditions. Maintenance of anhydrous conditions during crystallization is not essential but is to avoid the incomplete crystallization of some acid addition salts.

Crystallization may be carried out by commonly known methods such as cooling, addition of an anti-solvent, seeding and partial removal of the solvent or a combination thereof. The fractional crystallization may preferably be carried out at about 0°C to 45°C and more preferably at about 0°C to 35°C.

The acid addition salts of formulas I and II are novel and also form the part of the invention.

The preferred acid addition salts of formula I prepared according to the present invention are hydrogen halides, hydrogen sulfates, sulfates and sulfonic acid salts.

More preferred acid addition salts of formula I are hydrogen halides such as hydrogen chloride, hydrogen iodide and hydrogen bromide, still more preferred salt being hydrogen chloride salt.

Step (a) and (b) can be performed in the same solvent or different solvent. Even though the step (a) and (b) can be performed in different solvents, it is preferred to carry out the salt formation step and fractional crystallization in the same solvent in order to simplify the process.

Acid addition salts can also be prepared from the reaction mass obtained as a part of the synthesis of the compounds of formula II.

The crystalline acid addition salts of the compound of formulas 1 & II are novel.

The more preferred acid addition salts of compound of formula I prepared according to the present invention are hydrogen halide addition salts of formula III:

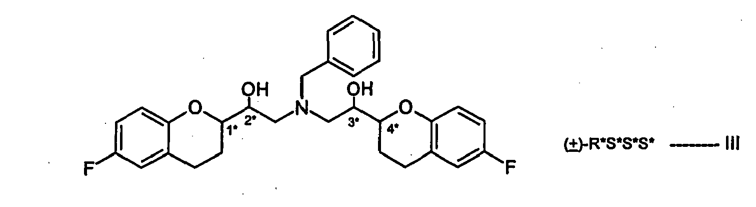

Still more preferred hydrogen halide addition salt of formula III is hydrogen chloride salt.

The process described above may also be used as a purification method for the removal of the undesired diastereomeric pair from the desired diastereomeric pair by basifying the acid addition salt of compound of formula I contaminated with undesired diastereomeric pair and then following the process steps (a) and (b) described above. The purification can be performed till the desired diastereomeric purity level is attained.

The compounds of formula II may be obtained by the methods known in the art. Thus, for example, the compounds of formula II are obtained by the process described in EP Patent No. 0145067 and EP Patent No. 0334429.

The acid addition salts of formula I are intermediates for preparing nebivolol and pharmaceutical acceptable salts thereof and can be converted into nebivolol by basifying with a base, removing the protecting group 'P' by the processes known in the art and optionally converting nebivolol into a pharmaceutically acceptable salt. The pharmaceutically acceptable salts were' described in U.S. patent No. 5,759,580 and incorporated herein by reference. The selection of the base is not critical but may be selected from hydroxides, carbonates and bicarbonates of alkaline metals; ammonia and amines. The amine base may be primary amine such as methylamine or ethylamine; secondary amine such as diethylamine or dimethylamine; and tert-amine such as triethylamine, trimethylamine or dimethylaminopyridine. The basification may be carried out in water or in an organic solvent or a mixture thereof. The organic solvents used here may be selected from the group consisting of C₁ to C₅ - alcohols, C₃ to C₈ -ketones, C₂ to C₈ -esters, acetonitrile, tetrahydrofuran, dimethylformamide, dimethylsulfoxide, dioxane, aromatic hydrocarbons, C₁ to C₅ -halogenated hydrocarbons and C₂ to C₈ -ethers and a mixture thereof. Preferable alcoholic solvents are methanol, ethanol, propanol and isopropyl alcohol; preferable ketonic solvents are acetone, methyl ethyl ketone, methyl isobutyl ketone, methyl tert-butyl ketone and diethyl ketone; preferable ester solvents are ethyl acetate, methyl acetate, isopropyl acetate, tert-butyl methyl acetate and ethyl formate; preferable aromatic hydrocarbon solvents are benzene, toluene and xylene; preferable halogenated hydrocarbon solvents are methylene chloride, chloroform, carbontetrachloride and ethylene dichloride; and preferable ether solvents are tert-butyl methyl ether and diethyl ether. Most preferable organic solvents are methanol, ethanol, propanol, isopropyl alcohol, acetone, methyl ethyl ketone, methyl isobutyl ketone, methyl tert-butyl ketone, diethyl ketone, acetonitrile, dimethylformamide, dimethylsulfoxide and tetrahydrofuran. Thus, for example, if P is benzyl then catalytic hydrogenation using hydrogenation catalyst such as palladium or platinum on carbon may be used for the de-protection and if P is allyl, then reaction with an appropriate noble metal compound such as PdCl₂ or Rh[P(C₆H₅)₃]Cl may be carried out.

The hydrogenation is carried out in a solvent. The selection of the solvent is not critical and may be selected from the group consisting of C₁ to C₅ - alcohols, C₃ to, C₈ -ketones, C₂ to C₈ -esters, acetonitrile, tetrahydrofuran, dimethylformamide, dimethylsulfoxide, dioxane, aromatic hydrocarbons, C₁ to C₅ -halogenated hydrocarbons and C₂ to C₈ -esthers and a mixture thereof. Preferable alcoholic solvents are methanol, ethanol, propanol and isopropyl alcohol; preferable ketonic solvents are acetone, methyl ethyl ketone, methyl isobutyl ketone, methyl tert-butyl ketone and diethyl ketone; preferable ester solvents are ethyl acetate, methyl acetate, isopropyl acetate, tert-butyl methyl acetate and ethyl formate; preferable aromatic hydrocarbon solvents are benzene, toluene and xylene; preferable halogenated hydrocarbon solvents are methylene chloride, chloroform, carbontetrachloride and ethylene dichloride; and preferable ether solvents are tert-butyl methyl ether and diethyl ether. Most preferable organic solvents are methanol, ethanol, propanol, isopropyl alcohol, acetone, methyl ethyl ketone, methyl isobutyl ketone; methyl tert-butyl ketone, diethyl ketone, acetonitrile, dimethylformamide, dimethylsulfoxide and tetrahydrofuran.

'Essentially anhydrous condition' refers to the water content less than 10%, preferably less than 5% and more preferably less than 2% of the total mass by weight.

In a preferred process (±)-[1S*(R*)]- (or (±)-[1S*(S*)])-6-fluoro-3,4-dihydro-α-[[(phenylmethyl)amino]methyl]-2H-1-benzopyran-2-methanol is reacted with (±)-[1S*(S*)]- (or (±)-[1S*(R*)])-6-fluoro-3,4-dihydro-2-oxiranyl-2H-1-benzopyran in an C₁ to C₅ -alcohol or C₃ to C₈ -ketone solvent to obtain (±)-[2R*[1S*,5S*(S*)]]+[2R*[1S*,5R*(R*)]]-α,α'-[phenylmethyliminobis(methylene)] bis[6-fluoro-3,4-dihydro-2H-1-benzopyran-2-methanol], hydrogen, halide, sulfate or hydrogen sulfate is added to the reaction mass, crystallization is performed at about 0°C to 45°C, the separated solid is filtered to obtain the corresponding salt of (±)-[2R*[1S*,5S*(S*)]]-α,α'-[phenylmethyliminobis(methylene)]bis[6-fluoro-3,4-dihydro-2H-1-benzopyran-2-methanol], the salt is basified in a solvent to obtain (±)-[2R*[1S*, 5S*(S*)]]-α,α'-[phenylmethyliminobis(methylene)]bis[6-fluoro-3,4-dihydro-2H-1-benzlpyran-2-methanol] free base and the free base is subjected to catalytic hydrogenation with hydrogen using palladium on carbon as catalyst.

The addition of hydrohalide may be performed by passing hydrogen halide gas, e.g., HCl (g) to the reaction mass or adding hydrogen halide dissolved in a solvent to the reaction mass.

Unless otherwise specified, the alkyl portion of the C₁ to C₅ -alcohol, C₃ to C₈ -ketone, C₂ to C₈ -ester, C₁ to C₅ -halogenated hydrocarbon and C₂ to C₈ ether used can be straight or branch, unsubstituted or substituted with for example alkoxy, halogen, nitro, cyano or hydroxy groups.

The invention will now be further described by the following examples, which are illustrative rather than limiting.

### Example 1

The solution of benzyl amine (14.89 gm) in ethanol (90 ml) is added to a mixture of (±)-[1S*(R*)]-6-fluoro-3,4-dihydro-2-oxiranyl-2H-1-benzopyran (9 gm) and ethanol (90 ml) drop wise at reflux temperature for 15 minutes. The temperature of the reaction mixture is raised to reflux and maintained for 5 hours at reflux temperature. Then ethanol is distilled off under vacuum at 50°C. To this residue diisopropyl ether (50 ml) added and stirred for 30 minutes at 0 - 5°C. Then the separated solid is filtered, washed with chilled diisopropylether and dried to give 8.5 gm of (±)-[1S*(R*)]-6-fluoro-3,4-dihydro-α-[[(phenylmethyl) amino]methyl]-2H-1-benzopyran-2-methanol (HPLC purity: 97%).

### Example 2

The mixture of (±)-[1S*(R*)]-6-fluoro-3,4-dihydro-α-[[(phenylmethyl) amino]methyl]-2H-1-benzopyran-2-methanol (100 gm, obtained in example 1), (±)-[1S*(S*)]-6-fluoro-3,4-dihydro-2-oxiranyl-2H-1-benzopyran (90 gm) and ethanol (2000 ml) is heated to reflux temperature and stirred for 8 hours at the same temperature to obtain (±)-[2R*[1S*, 5S*(S*)]]+[2R*[1S*,5R*(R*)]]-α,α'-[phenylmethyliminobis(methylene)]bis[6-fluoro-3,4-dihydro-2H-1-benzopyran-2-methanol] ((±)-[2R*[1S*, 5S*(S*)]] to (±)-[2R*[1S*,5R*(R*)]] ratio is 1 : 1.1). Then the reaction mass is cooled to 10°C, the pH is adjusted to 2 with HCl gas and stirred for 45 minutes at 25°C to 30°C. Then the separated solid is filtered and dried to give 80.7 gm of (±)-[2R*[1S*, 5S*(S*)]]-α,α'-[phenylmethyliminobis (methylene)]bis[6-fluoro-3,4-dihydro-2H-1-benzopyran-2-methanol] hydrochloride salt (HPLC purity 99.0%; (±)-[2R*[1S*, 5S*(S*)]] to (±)-[2R*[1S*,5R*(R*)]] ratio is 99.4 : 0.6).

### Example 3

The mixture of 10% NaHCO₃ solution (800 ml) and (±)-[2R*[1S*, 5S*(S*)]]-α,α'-[phenylmethyliminobis(methylene)]bis[6-fluoro-3,4-dihydro-2H-1-benzopyran-2-methanol] hydrochloride salt (80.7 gm) obtained above is stirred for 15 minutes and then extracted twice with ethyl acetate (1600 ml). Then the organic layer is washed with water (800 ml) and 20% sodium chloride solution (400 ml) and then distilled off the solvent to give 38.4 gm of (±)-[2R*[1S*, 5S*(S*)]]-α,α'-[phenylmethyliminobis(methylene)]bis[6-fluoro-3,4-dihydro-2H-1-benzopyran-2-methanol] residue.

The mixture of (±)-[2R*[1S*, 5S*(S*)]]-α,α'-[phenylmethyliminobis (methylene)]bis[6-fluoro-3,4-dihydro-2H-1-benzopyran-2-methanol] residue (38.4 gm, obtained above, 10% palladium on charcoal (10 gm) and ethanol (1300ml) is taken into a hydrogenation flask and subjected to hydrogenation under a hydrogen gas pressure of 2.5 kg/cm² for 3 hours. Then the reaction mixture is filtered on hi-flo and washed with ethanol. The solvent is distilled off, acetonitrile (200 ml) is added and stirred for 10 minutes. Then the separated solid is filtered and washed with acetonitrile to give 10 gm of (±)-[2R*[1S*,5S*(S*)]]-α,α'-[iminobis(methylene)]bis[6-fluoro-3,4-dihydro-2H-1-benzopyran-2-methanol] (Nebivolol) (HPLC purity: 99.3%).

### Example 4

Nebivolol (10 gm) obtained in example 3 is dissolved in the mixture of methylene dichloride (150 ml) and ethanol (100 ml) at 45°C and then cooled to 10°C. Then pH of the solution is adjusted to 2 with HCl gas, stirred for 10 minutes and distilled the solvent. Then the solid is filtered and washed with acetone to give 7 gm of Nebivolol hydrochloride salt (HPLC purity: 99.8%).

## Claims

1. A process for preparing acid additional salts of compounds of formula I: 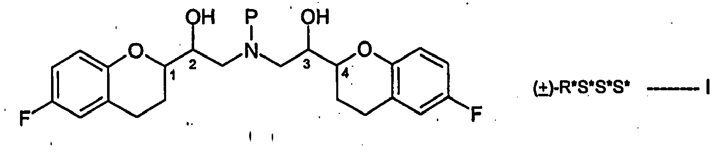 wherein
P is -allyl or 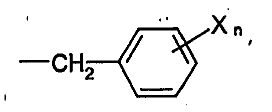 wherein
X each independently is halo, nitro or C₁-C₃ alkyl and n is 0 - 5;
which comprises:
a) treating a mixture containing racemic diastereomers of a compound of formula II: 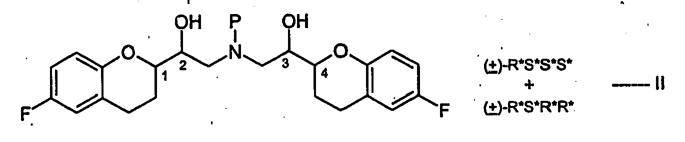 wherein P is as defined in formula I;
with a suitable acid to form the corresponding acid addition salt;
b) subjecting the acid addition salt obtained in step (a) to the fractional crystallization from an alcoholic solvent, ketonic solvent, acetonitrile, dimethylformamide, dimethylsulfoxide and tetrahydrofuran or mixture there of to obtain the diastereomeric pair of compound of formula I.

2. The process according to claim 1, wherein the alcoholic solvent is selected from the group consisting of C₁ to C₅ -alcohols.

3. The process according to claim 2, wherein the alcoholic solvent is selected from methanol, ethanol, propanol and isopropyl alcohol.

4. The process according to claim 3, wherein the alcoholic solvent is ethanol.

5. The process according to claim 1, wherein the ketonic solvent is selected from the group consisting of C₃ to C₈ -ketones.

6. The process according to claim 5, wherein the ketonic solvent is selected from acetone, methyl isobutyl ketone and methyl tert-butyl ketone.

7. The process according to claim 6, wherein the ketonic solvent is acetone.

8. The process according to claim 1, wherein the acid addition salt is prepared by treating the mixture containing compounds of formula II with the corresponding acid in a solvent.

9. The process according to claim 8, wherein the acid is inorganic acid or an organic acid.

10. The process according to claim 9, wherein the inorganic acid is a hydrogen halide, nitric acid or phosphoric acid.

11. The process according to claim 10, wherein the hydrogen halide is hydrogen chloride.

12. The process according to claim 9, wherein the organic acid is a carboxylic acid or a sulfonic acid.

13. The process according to claim 12, wherein the carboxylic acid is selected from acetic acid, propanoic acid, formic acid, hydroxyacetic acid, 2-hydroxy propanoic acid, 2-oxopropanoic acid, propane dioic acid, butanedioic acid, (Z)-2-butenedioic acid, (E)-2-butenedioic acid and 2-hydroxy butanedioic acid.

14. The process according to claim 12, wherein the sulfonic acid is selected from methane sulfonic acid, toluene sulfonic acid and benzene sulfonic acid.

15. The process according to claim 8, wherein the solvent is an organic solvent.

16. The process according to claim 15, wherein the organic solvent is selected from the group consisting of C₁ to C₅ -alcohols, C₃ to C₈ -ketones, C₂ to C₈-esters, acetonitrile, tetrahydrofuran, dimethylformamide, dimethylsulfoxide, dioxane, aromatic hydrocarbons, C₁ to C₅ -halogenated hydrocarbons and C₂ to C₈ -ethers and mixture thereof.

17. The process according to claim 16, wherein the alcoholic solvents are methanol, ethanol, propanol and isopropyl alcohol; ketonic solvents are acetone, methyl ethyl ketone, methyl isobutyl ketone, methyl tert-butyl ketone and diethyl ketone; ester solvents are ethyl acetate, methyl acetate, isopropyl acetate, tert-butyl methyl acetate and ethyl formate; aromatic hydrocarbon solvents are benzene, toluene and xylene; halogenated hydrocarbon solvents are methylene chloride, chloroform, carbontetrachloride and ethylene dichloride; and ether solvents are tert-butyl methyl ether and diethyl ether.

18. The process according to claim 16, wherein the organic solvent is selected from methanol, ethanol, propanol, isopropyl alcohol, acetone, methyl ethyl ketone, methyl isobutyl ketone, methyl tert-butyl ketone, diethyl ketone, acetonitrile, dimethylformamide, dimethylsulfoxide and tetrahydrofuran.

19. The process according to claim 18, wherein the organic solvent is methanol, ethanol or acetone.

20. The process according to claim 1, wherein the fractional crystallization is carried out in anhydrous condition.

21. The process according to claims 1 and 20, wherein the crystallization is carried out by cooling, addition of anti-solvents, seeding or partial removal of the solvents or combination thereof.

22. The process according to claim 21, wherein the crystallization is carried out at about 0°C to 45°C.

23. The process according to claim 22, wherein the crystallization is carried out at about 0°C to 35°C.

24. The process according to claim 1, wherein the steps (a) and (b) are performed in the same solvent or different solvent.

25. The process according to claim 24, whereon the steps (a) and (b) are performed in different solvents.

26. The process according to claim 1, wherein the acid addition salts of formula I are prepared from the reaction mass obtained as a part of the synthesis of the compounds of formula II.

27. The process according to claims 1 and 26, wherein the acid addition salts of compound of formula I prepared according to the present invention are hydrogen halide addition salts of formula III:

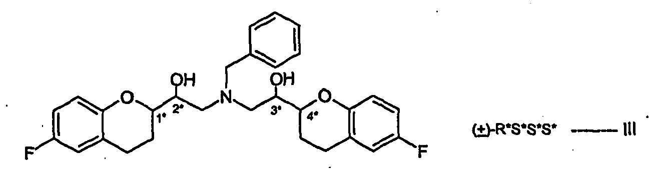

28. The process according to claim 27, wherein the hydrogen halide salt of formula III is hydrogen chloride salt.

29. A process for the preparation of nebivolol or a pharmaceutically acceptable salt thereof, which comprises the steps of:
a) reacting 6-fluoro-3,4-dihydro-α-[[(phenylmethyl)amino]methyl]-2H-1-benzo pyran-2-methanol with 6-fluoro-3,4-dihydro-2-oxiranyl-2H-1-benzopyran in an C₁ to C₅ -alcohol or C₃ to C₈ -ketone solvent to produce (±)-[2R*[1S*,5S*(S*)]]+[2R*[1S*,5R*(R*)]]-α,α'-[phenylmethyliminobis(methyle , ne)]bis[6-fluoro-3,4-dihydro-2H-1-benzopyran-2-methanol];
b) treating (±)-[2R*[1S*,5S*(S*)]]+[2R*[1S*,5R*(R*)]]-α,α'-[phenylmethylimino bis(methylene)]bis[6-fluoro-3,4-dihydro-2H-1-benzopyran-2-methanol] produced in step (a) with hydrogen halide, sulfate or hydrogen sulfate; subjecting to fractional crystallization at about 0°C to 45°C and filtering the separated solid to produce corresponding salt of (±)-[2R*[1S*,5S*(S*)]]-α,α'-[phenylmethyliminobis(methylene)]bis[6-fluoro-3,4-dihydro-2H-1-benzopyran -2-methanol];
c) basifying the salt of (±)-[2R*[1S*,5S*(S*)]]-α,α'-[phenylmethylimino bis(methylene)]bis[6-fluoro-3,4-dihydro-2H-1-benzopyran-2-methanol] produced in step(b) in a solvent to produce (±)-[2R*[1S*,5S*(S*)]]-α,α'-[phenylmethyliminobis(methylene)]bis[6-fluoro-3,4-dihydro-2H-1-benzopyran -2-methanol] free base; and
d) subjecting (±)-[2R*[1S*,5S*(S*)]]-α,α'-[phenylmethyliminobis(methylene)]bis [6-fluoro-3,4-dihydro-2H-1-benzopyran -2-methanol] free base to catalytic hydrogenation with hydrogen using palladium on carbon as catalyst to obtain nebivolol optionally converting to the pharmaceutically acceptable salt.

30. The process according to claim 29, wherein the alcoholic solvent is selected from the group consisting of C₁ to C₅ -alcohols.

31. The process according to claim 30, wherein the alcoholic solvent is selected from methanol, ethanol, propanol and isopropyl alcohol.

32. The process according to claim 31, wherein the alcoholic solvent is ethanol.

33. The process according to claim 29, wherein the ketonic solvent is selected from the group consisting of C₃ to C₈ -ketones.

34. The process according to claim 33, wherein the ketonic solvent is selected from acetone, methyl isobutyl ketone and methyl tert-butyl ketone.

35. The process according to claim 34, wherein the ketonic solvent is acetone.

36. The process according to claim 29, wherein the hydrogen halide is hydrogen chloride.

37. The process according to claim 36, wherein the treatment in step (b) is carried out by passing hydrogen chloride gas to the mass containing (±)-[2R*[1S*,5S*(S*)]]+[2R*[1S*,5R*(R*)]]-α,α'-[phenylmethyliminobis(methyl ene)]bis[6-fluoro-3,4-dihydro-2H-1-benzopyran-2-methanol] (or) by adding a solution of hydrogen chloride in a solvent to the mass containing (±)-[2R*[1S*,5S*(S*)]]+[2R*[1S*,5R*(R*)]]-α,α'-[phenylmethyliminobis(methyl ene)]bis[6-fluoro-3,4-dihydro-2H-1-benzopyran-2-methanol].

38. Acid addition salts of formulas I and II: 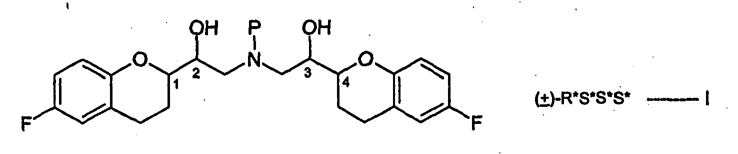 wherein
P is -allyl or 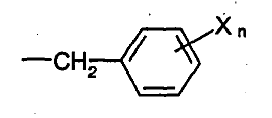 wherein X each independently is halo, nitro or C₁-C₃ alkyl and n is 0 - 5; 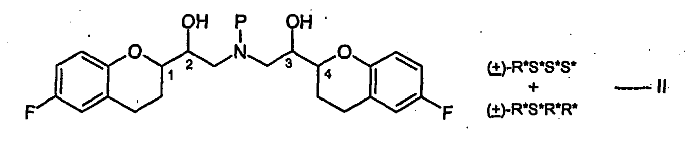 wherein P is as defined in formula I.

39. The acid addition salts of claim 38, wherein the said salts are hydrogen halides, hydrogen sulfates, sulfates and sulfonic acid salts.

40. The acid addition salts of claim 39, wherein the said salts are hydrogen halides.

41. The acid addition salts of claim 40, wherein the said salts are hydrogen chloride, hydrogen iodide and hydrogen bromide.

42. The acid addition salts of claim 41, wherein the said salt is hydrogen chloride.

## Patentansprüche

1. Verfahren für die Herstellung von sauren Additionssalzen von Verbindungen der Formel I: 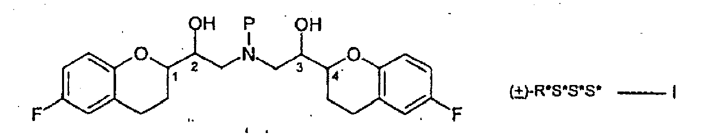 wobei
P -allyl oder 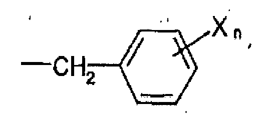 ist,
wobei
X jeweils unabhängig Halo, Nitro oder C₁-C₃-Alkyl ist und n 0 - 5 beträgt;
das Folgendes umfasst:
a) das Behandeln einer Mischung, die racemische Diastereomere einer Verbindung der Formel II: 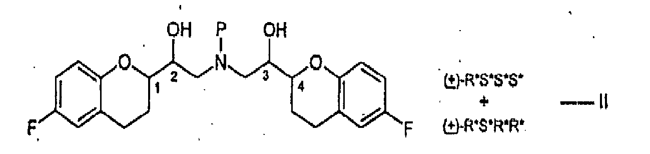 enthält, wobei P die in Formel I angegebene Definition aufweist;
mit einer geeigneten Säure unter Bildung des entsprechenden sauren Additionssalzes;
b) das Unterwerfen des in Schritt (a) erhaltenen sauren Additionssalzes der fraktionierten Kristallisation aus einem alkoholischen Lösungsmittel, ketonischen Lösungsmittel, Acetonitril, Dimethylformamid, Dimethylsulfoxid und Tetrahydrofuran oder Mischungen derselben, um das diastereomere Paar der Verbindung der Formel I zu erhalten.

2. Verfahren nach Anspruch 1, wobei das alkoholische Lösungsmittel aus der Gruppe ausgewählt wird bestehend aus C₁- bis C₅-Alkoholen.

3. Verfahren nach Anspruch 2, wobei das alkoholische Lösungsmittel unter Methanol, Ethanol, Propanol und Isopropylalkohol ausgewählt wird.

4. Verfahren nach Anspruch 3, wobei das alkoholische Lösungsmittel Ethanol ist.

5. Verfahren nach Anspruch 1, wobei das ketonische Lösungsmittel aus der Gruppe ausgewählt wird bestehend aus C₃- bis C₈-Ketonen.

6. Verfahren nach Anspruch 5, wobei das ketonische Lösungsmittel unter Aceton, Methylisobutylketon und Methyl-tert.-butylketon ausgewählt wird.

7. Verfahren nach Anspruch 6, wobei das ketonische Lösungsmittel Aceton ist.

8. Verfahren nach Anspruch 1, wobei das saure Additionssalz durch Behandeln der Mischung, die die Verbindungen der Formel II enthält, mit der entsprechenden Säure in einem Lösungsmittel hergestellt wird.

9. Verfahren nach Anspruch 8, wobei die Säure anorganische Säure oder eine organische Säure ist.

10. Verfahren nach Anspruch 9, wobei die anorganische Säure ein Wasserstoffhalogenid, Salpetersäure oder Phosphorsäure ist.

11. Verfahren nach Anspruch 10, wobei das Wasserstoffhalogenid Wasserstoffchlorid ist.

12. Verfahren nach Anspruch 9, wobei die organische Säure ein Carbonsäure oder eine Sulfonsäure ist.

13. Verfahren nach Anspruch 12, wobei die Carbonsäure unter Essigsäure, Propionsäure, Ameisensäure, Hydroxyessigsäure, 2-Hydroxypropionsäure, 2-Oxopropionsäure, Propandisäure, Butandisäure, (Z)-2-Butendisäure, (E)-2-Butendisäure und 2-Hydroxybutandisäure ausgewählt wird.

14. Verfahren nach Anspruch 12, wobei die Sulfonsäure unter Methansulfonsäure, Toluolsulfonsäure und Benzolsulfonsäure ausgewählt wird.

15. Verfahren nach Anspruch 8, wobei das Lösungsmittel ein organisches Lösungsmittel ist.

16. Verfahren nach Anspruch 15, wobei das organische Lösungsmittel aus der Gruppe ausgewählt ist bestehend aus C₁- bis C₅-Alkoholen, C₃- bis C₈-Ketonen, C₂- bis C₈-Estern, Acetonitril, Tetrahydrofuran, Dimethylformamid, Dimethylsulfoxid, Dioxan, aromatischen Kohlenwasserstoffen, halogenierten C₁- bis C₅-Kohlenwasserstoffen und C₂- bis C₈-Ethern und Mischungen derselben.

17. Verfahren nach Anspruch 16, wobei die alkoholischen Lösungsmittel Methanol, Ethanol, Propanol und Isopropylalkohol sind; die ketonischen Lösungsmittel Aceton, Methylethylketon, Methylisobutylketon, Methyl-tert.-butylketon und Diethylketon sind; die Esterlösungsmittel Ethylacetat, Methylacetat, Isopropylacetat, tert.-Butylmethylacetat und Ethylformiat sind; die aromatischen Kohlenwasserstofflösungsmittel Benzol, Toluol und Xylol sind; die halogenierten Kohlenwasserstofflösungsmittel Methylenchlorid, Chloroform, Tetrachlorkohlenstoff und Ethylendichlorid sind; und die Etherlösungsmittel tert.-Butylmethylether und Diethylether sind.

18. Verfahren nach Anspruch 16, wobei das organische Lösungsmittel unter Methanol, Ethanol, Propanol, Isopropylalkohol, Aceton, Methylethylketon, Methylisobutylketon, Methyl-tert.-butylketon, Diethylketon, Acetonitril, Dimethylformamid, Dimethylsulfoxid und Tetrahydrofuran ausgewählt wird.

19. Verfahren nach Anspruch 18, wobei das organische Lösungsmittel Methanol, Ethanol oder Aceton ist.

20. Verfahren nach Anspruch 1, wobei die fraktionierte Kristallisation unter wasserfreien Bedingungen durchgeführt wird.

21. Verfahren nach Anspruch 1 und 20, wobei die Kristallisation durch Kühlen, Zusetzen von Antilösungsmitteln, Impfen oder teilweises Entfernen der Lösungsmittel oder Kombinationen davon durchgeführt wird.

22. Verfahren nach Anspruch 21, wobei die Kristallisation bei etwa 0 °C bis 45 °C durchgeführt wird.

23. Verfahren nach Anspruch 22, wobei die Kristallisation bei etwa 0 °C bis 35 °C durchgeführt wird.

24. Verfahren nach Anspruch 1, wobei die Schritte (a) und (b) in demselben Lösungsmittel oder in verschiedenen Lösungsmitteln durchgeführt werden.

25. Verfahren nach Anspruch 24, wobei die Schritte (a) und (b) in verschiedenen Lösungsmitteln durchgeführt werden.

26. Verfahren nach Anspruch 1, wobei die sauren Additionssalze der Formel I aus der Reaktionsmasse hergestellt werden, die als Teil der Synthese der Verbindungen der Formel II erhalten werden.

27. Verfahren nach den Ansprüchen 1 und 26, wobei die sauren Additionssalze der Verbindung der Formel I, die erfindungsgemäß hergestellt werden, Wasserstoffhalogenid-Additionssalze der Formel III 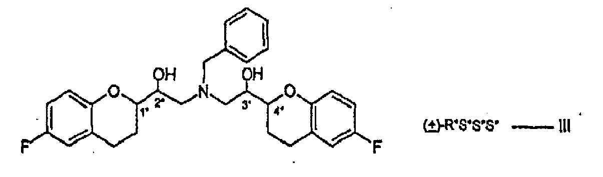 sind.

28. Verfahren nach Anspruch 27, wobei das Wasserstoffhalogenidsalz der Formel III Chlorwasserstoffsalz ist.

29. Verfahren für die Herstellung von Nebivolol oder einem pharmazeutisch akzeptablen Salz desselben, das folgende Schritte umfasst:
a) das Reagieren von 6-Fluor-3,4-dihydro-α-[[phenylmethyl)amino]methyl]-2H-1-benzopyran-2-methanol mit 6-Fluor-3,4-dihydro-2-oxiranyl-2H-1-benzopyran in einem C₁- bis C₅-Alkohol- oder C₃-bis C₈-Ketonlösungsmittel unter Bildung von (±)-[2R*[1S*,5S*(S*)]]+[2R*[1S*,5R*(R*)]]-α,α'-[phenylmethyliminobis(methylen)]bis[6-fluor-3,4-dihydro-2H-1-benzopyran-2-methanol];
b) das Behandeln von (±)-[2R*[1S*,5S*(S*)]]+[2R*[1S*,5R*(R*)]]-α,α'-[phenylmethyliminobis(methylen)]bis[6-fluor-3,4-dihydro-2H-1-benzopyran-2-methanol], das in Schritt (a) hergestellt worden ist, mit Wasserstoffhalogenid, -sulfat oder Wasserstoffsulfat; Unterwerfen der fraktionierten Kristallisation bei etwa 0 °C bis 45 °C und Filtrieren des abgetrennten Feststoffs unter Bildung des entsprechenden Salzes von (±)-[2R*[1S*,5S*(S*)]]-α,α-[phenylmethyliminobis(methylen)]bis[6-fluor-3,4-dihydro-2H-1-benzopyran-2-methanol];
c) das Alkalisieren des Salzes von (±)-[2R*[1S*,5S*(S*)]]-α,α'-[phenylmethyliminobis(methylen)]bis[6-fluor-3,4-dihydro-2H-1-benzopyran-2-methanol], das in Schritt (b) hergestellt worden ist, in einem Lösungsmittel unter Bildung von (±)-[2R*[1S*,55*(S*)]]-α,α'-[phenylmethyliminobis(methylen)]bis[6-fluor-3,4-dihydro-2H-1-benzopyran-2-methanol] als freie Base; und
d) das Unterwerfen der freien (±)-[2R*[1S*,5S*(S*)]]-α,α'-[phenylmethyliminobis(methylen)]bis[6-fluor-3,4-dihydro-2H-1-benzopyran-2-methanol]-Base einer katalytischen Hydrierung mit Wasserstoff unter Anwendung von Palladium auf Kohlenstoff als Katalysator, um Nebivolol zu erhalten und wahlweise Umwandeln desselben zum pharmazeutisch akzeptablen Salz.

30. Verfahren nach Anspruch 29, wobei das alkoholische Lösungsmittel aus der Gruppe ausgewählt wird bestehend aus C₁- bis C₅-Alkoholen.

31. Verfahren nach Anspruch 30, wobei das alkoholische Lösungsmittel unter Methanol, Ethanol, Propanol und Isopropylalkohol ausgewählt wird.

32. Verfahren nach Anspruch 31, wobei das alkoholische Lösungsmittel Ethanol ist.

33. Verfahren nach Anspruch 29, wobei das ketonische Lösungsmittel aus der Gruppe ausgewählt wird bestehend aus C₃- bis C₈-Ketonen.

34. Verfahren nach Anspruch 33, wobei das ketonische Lösungsmittel unter Aceton, Methylisobutylketon und Methyl-tert.-butylketon ausgewählt wird.

35. Verfahren nach Anspruch 34, wobei das ketonische Lösungsmittel Aceton ist.

36. Verfahren nach Anspruch 29, wobei das Wasserstoffhalogenid Chlorwasserstoff ist.

37. Verfahren nach Anspruch 36, wobei die Behandlung in Schritt (b) durch Hinzuführen von Chlorwasserstoffgas zu der Masse, die (±)-[2R*[1S*,5S*(S*)]]+[2R*[1S*,5R*(R*)]]-α,α'-[phenylmethyliminobis(methylen)]bis[6-fluor-3,4-dihydro-2H-1-benzopyran-2-methanol] enthält (oder) durch Hinzufügen einer Lösung von Chlorwasserstoff in einem Lösungsmittel zu der Masse, die (±)-[2R*[1S*,5S*(S*)]]+[2R*[1S*,5R*(R*)]]-α,α'-[phenylmethyliminobis(methylen)]bis[6-fluor-3,4-dihydro-2H-1-benzopyran-2-methanol] enthält.

38. Saure Additionssalze der Formeln I und II: 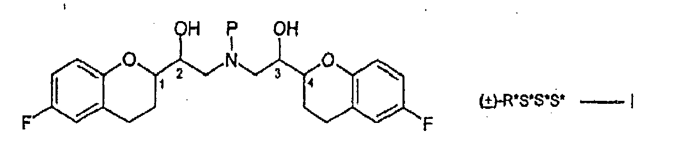 wobei
P -allyl oder 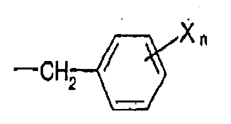 ist, wobei X jeweils unabhängig Halo, Nitro oder C₁-C₃-Alkyl ist und n 0 - 5 beträgt; 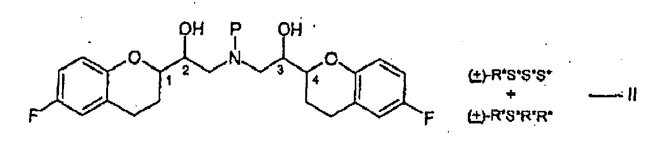 wobei P die in Formel I angegebene Definition besitzt.

39. Saure Additionssalze nach Anspruch 38, wobei die Salze Wasserstoffhalogenide, Wasserstoffsulfate, Sulfate oder Sulfonsäuresalze sind.

40. Saure Additionssalze nach Anspruch 39, wobei die Salze Wasserstoffhalogenide sind.

41. Saure Additionssalze nach Anspruch 40, wobei die Salze Wasserstoffchlorid, Wasserstoffiodid und Wasserstoffbromid sind.

42. Saure Additionssalze nach Anspruch 41, wobei das Salz Wasserstoffchlorid ist.

## Revendications

1. Procédé pour la préparation de sels d'addition d'acide de composés de formule I : 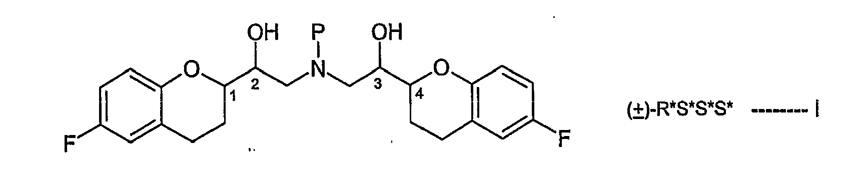 dans laquelle
P est -allyle ou 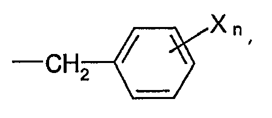 où
chaque X est indépendamment un groupe halo, nitro ou alkyle en C₁-C₃ et n est 0-5 ;
lequel comprend :
a) le traitement d'un mélange contenant des diastéréoisomères racémiques d'un composé de formule II : 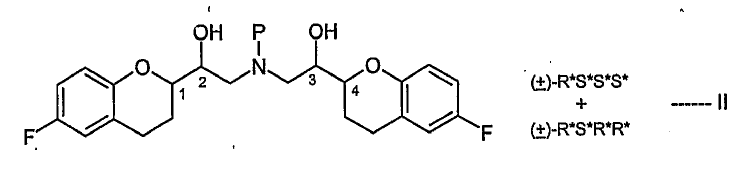 dans laquelle P est tel que défini dans la formule I ;
avec un acide approprié pour former le sel d'addition d'acide correspondant ;
b) le fait de soumettre le sel d'addition d'acide obtenu dans l'étape (a) à la cristallisation fractionnée dans un solvant alcoolique, un solvant cétonique, l'acétonitrile, le diméthylformamide, le diméthylsulfoxyde et le tétrahydrofurane ou un mélange de ceux-ci pour obtenir la paire de diastéréoisomères du composé de formule I.

2. Procédé selon la revendication 1, dans lequel le solvant alcoolique est choisi dans le groupe constitué par les alcools en C₁ à C₅.

3. Procédé selon la revendication 2, dans lequel le solvant alcoolique est choisi parmi le méthanol, l'éthanol, le propanol et l'alcool isopropylique.

4. Procédé selon la revendication 3, dans lequel le solvant alcoolique est l'éthanol.

5. Procédé selon la revendication 1, dans lequel le solvant cétonique est choisi dans le groupe constitué par les cétones en C₃ à C₈.

6. Procédé selon la revendication 5, dans lequel le solvant cétonique est choisi parmi l'acétone, la méthylisobutylcétone et la méthyl-tert-butylcétone.

7. Procédé selon la revendication 6, dans lequel le solvant cétonique est l'acétone.

8. Procédé selon la revendication 1, dans lequel le sel d'addition d'acide est préparé par traitement du mélange contenant des composés de formule II avec l'acide correspondant dans un solvant.

9. Procédé selon la revendication 8, dans lequel l'acide est un acide inorganique ou un acide organique.

10. Procédé selon la revendication 9, dans lequel l'acide inorganique est un halogénure d'hydrogène, l'acide nitrique ou l'acide phosphorique.

11. Procédé selon la revendication 10, dans lequel l'halogénure hydrogène est le chlorure d'hydrogène.

12. Procédé selon la revendication 9, dans lequel l'acide organique est un acide carboxylique ou un acide sulfonique.

13. Procédé selon la revendication 12, dans lequel l'acide carboxylique est choisi parmi l'acide acétique, l'acide propanoïque, l'acide formique, l'acide hydroxyacétique, l'acide 2-hydroxypropanoïque, l'acide 2-oxopropanoïque, l'acide propanedioïque, l'acide butanedioïque, l'acide (Z)-2-butènedioïque, l'acide (*E*)-2-butènedioïque et l'acide 2-hydroxybutanedioïque.

14. Procédé selon la revendication 12, dans lequel l'acide sulfonique est choisi parmi l'acide méthanesulfonique, l'acide toluènesulfonique et l'acide benzènesulfonique.

15. Procédé selon la revendication 8, dans lequel le solvant est un solvant organique.

16. Procédé selon la revendication 15, dans lequel le solvant organique est choisi dans le groupe constitué par les alcools en C₁ à C₅, les cétones en C₃ à C₈, les esters en C₂ à C₈, l'acétonitrile, le tétrahydrofurane, le diméthylformamide, le diméthylsulfoxyde, le dioxane, les hydrocarbures aromatiques, les hydrocarbures halogénés en C₁ à C₅ et les éthers en C₂ à C₈ et un mélange de ceux-ci.

17. Procédé selon la revendication 16, dans lequel les solvants alcooliques sont le méthanol, l'éthanol, le propanol et l'alcool isopropylique ; les solvants cétoniques sont l'acétone, la méthyléthylcétone, la méthylisobutylcétone, la méthyl-*tert*-butylcétone et la diéthylcétone ; les solvants esters sont l'acétate d'éthyle, l'acétate de méthyle, l'acétate d'isopropyle, l'acétate de *tert*-butylméthyle et le formiate d'éthyle ; les solvants hydrocarbures aromatiques sont le benzène, le toluène et le xylène ; les solvants hydrocarbures halogénés sont le chlorure de méthylène, le chloroforme, le tétrachlorure de carbone et le dichlorure d'éthylène ; et les solvants éthers sont l'oxyde de *tert*-butyle et de méthyle et l'oxyde de diéthyle.

18. Procédé selon la revendication 16, dans lequel le solvant organique est choisi parmi le méthanol, l'éthanol, le propanol, l'alcool isopropylique, l'acétone, la méthyléthylcétone, la méthylisobutylcétone, la méthyl-*tert*-butylcétone, la diéthylcétone, l'acétonitrile, le diméthylformamide, le diméthylsulfoxyde et le tétrahydrofurane.

19. Procédé selon la revendication 18, dans lequel le solvant organique est le méthanol, l'éthanol ou l'acétone.

20. Procédé selon la revendication 1, dans lequel la cristallisation fractionnée est effectuée à l'état anhydre.

21. Procédé selon les revendications 1 et 20, dans lequel la cristallisation est effectuée par refroidissement, ajout d'antisolvants, amorçage avec un germe cristallin ou élimination partielle des solvants ou des combinaisons de ces procédés.

22. Procédé selon la revendication 21, dans lequel la cristallisation est effectuée à environ 0 °C à 45 °C.

23. Procédé selon la revendication 22, dans lequel la cristallisation est effectuée à environ 0 °C à 35 °C.

24. Procédé selon la revendication 1, dans lequel les étapes (a) et (b) sont effectuées dans le même solvant ou un solvant différent.

25. Procédé selon la revendication 24, dans lequel les étapes (a) et (b) sont effectuées dans des solvants différents.

26. Procédé selon la revendication 1, dans lequel les sels d'addition d'acide de formule I sont préparés à partir de la masse réactionnelle obtenue en tant que partie de la synthèse des composés de formule II.

27. Procédé selon les revendications 1 et 26, dans lequel les sels d'addition d'acide du composé de formule I préparés selon la présente invention sont des sels d'addition d'halogénure d'hydrogène de formule III :

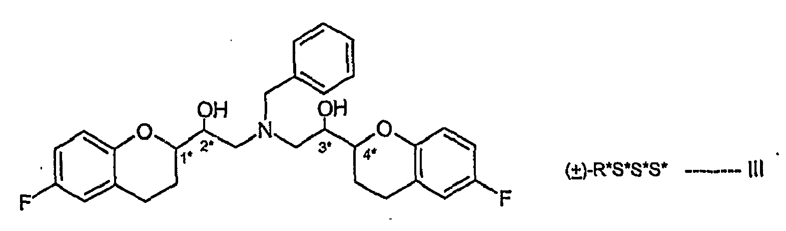

28. Procédé selon la revendication 27, dans lequel le sel d'halogénure d'hydrogène de formule III est un sel de chlorure d'hydrogène.

29. Procédé pour la préparation de nébivolol ou d'un sel pharmaceutiquement acceptable de celui-ci, lequel comprend les étapes consistant à :
a) faire réagir du 6-fluoro-3,4-dihydro-a-[[(phénylméthyl)amino]méthyl]-2*H*-1-benzopyran-2-méthanol avec du 6-fluoro-3,4-dihydro-2-oxiranyl-2*H*-1-benzopyrane dans un solvant alcool en C₁ à C₅ ou cétone en C₃ à C₈ pour produire du (±)-[2*R**[1*S**,5*S**(*S**)]]+[2*R**[1*S**,5*R**(*R**)]]-α,α'-[phénylméthyliminobis(méthylène)]bis[6-fluoro-3,4-dihydro-2H-1-benzopyran-2-méthanol] ;
b) traiter le (±)-[2*R**[1*S**,5*S**(*S**)]]+[2*R**[1*S**,5*R**(*R**)]]-α,α'-[phénylméthyliminobis(méthylène)]bis[6-fluoro-3,4-dihydro-2*H*-1-benzopyran-2-méthanol] produit dans l'étape (a) avec de l'halogénure d'hydrogène, du sulfate ou de l'hydrogénosulfate ; soumettre'à une cristallisation fractionnée à environ 0 °C à 45 °C et filtrer le solide séparé pour produire le sel correspondant de (±)-[2*R**[1*S**,5*S**(*S**)]]-α,α'-[phénylméthyliminobis(méthylène)]bis[6-fluoro-3,4-dihydro-2*H*-1-benzopyran-2-méthanol] ;
c) basifier le sel de (±)-[2*R**[1*S**,5*S**(*S**)]]-α,α'-[phénylméthyliminobis(méthylène)]bis[6-fluoro-3,4-dihydro-2*H*-1-benzopyran-2-méthanol] produit dans l'étape (b) dans un solvant pour produire la base libre de (±)-[2*R**[1*S**,5*S**(*S**)]]-α,α'-[phénylméthyliminobis(méthylène)]bis[6-fluoro-3,4-dihydro-2*H*-1-benzopyran-2-méthanol] ; et
d) soumettre la base libre de (±)-[2*R**[1*S**,5*S**(*S**)]]-α,α'-[phénylméthyliminobis(méthylène)]bis[6-fluoro-3,4-dihydro-2*H*-1-benzopyran-2-méthanol] à une hydrogénation catalytique avec de l'hydrogène à l'aide de palladium sur du carbone comme catalyseur pour obtenir du nébivolol et facultativement convertir en le sel pharmaceutiquement acceptable.

30. Procédé selon la revendication 29, dans lequel le solvant alcoolique est choisi dans le groupe constitué par les alcools en C₁ à C₅.

31. Procédé selon la revendication 30, dans lequel le solvant alcoolique est choisi parmi le méthanol, l'éthanol, le propanol et l'alcool isopropylique.

32. Procédé selon la revendication 31, dans lequel le solvant alcoolique est l'éthanol.

33. Procédé selon la revendication 29, dans lequel le solvant cétonique est choisi dans le groupe constitué par les cétones en C₃ à C₈.

34. Procédé selon la revendication 33, dans lequel le solvant cétonique est choisi parmi l'acétone, la méthylisobutylcétone et la méthyl-*tert*-butylcétone.

35. Procédé selon la revendication 34, dans lequel le solvant cétonique est l'acétone.

36. Procédé selon la revendication 29, dans lequel l'halogénure d'hydrogène est le chlorure d'hydrogène.

37. Procédé selon la revendication 36, dans lequel le traitement dans l'étape (b) est effectué par passage de chlorure d'hydrogène gazeux sur la masse contenant du (±)-[2*R**[1*S**,5*S**(*S**)]]+[2*R**[1*S**,5*R**(*R**)]]-α,α'-[phénylméthyliminobis(méthylène)]bis[6-fluoro-3,4-dihydro-2H-1-benzopyran-2-méthanol] (ou) par ajout d'une solution de chlorure d'hydrogène dans un solvant à la masse contenant du (±)-[2*R**[1*S**,5*S**(*S**)]]+[2*R**[1*S**,5*R**(*R**)]]-α,α'-[phénylméthyliminobis(méthylène)]bis[6-fluoro-3,4-dihydro-2*H*-1-benzopyran-2-méthanol].

38. Sels d'addition d'acide représentés par les formules I et II : 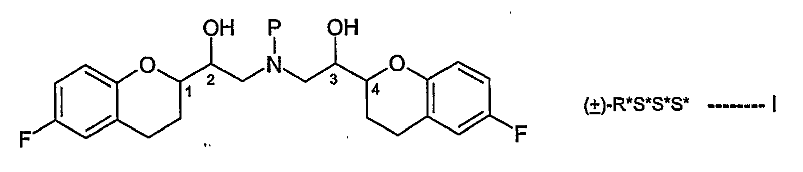 dans laquelle
P est -allyle ou 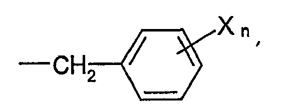 où
chaque X est indépendamment un groupe halo, nitro ou alkyle en C₁-C₃ et n est 0-5 ; 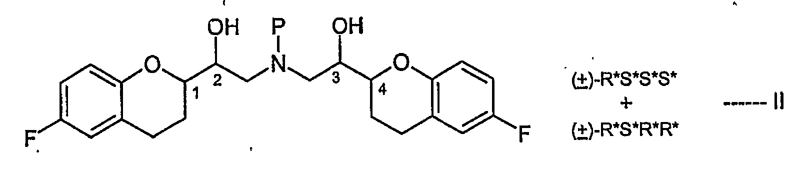 dans laquelle P est tel que défini dans la formule I.

39. Sels d'addition d'acide selon la revendication 38, lesdits sels étant des sels d'halogénure d'hydrogène, des hydrogénosulfates, des sulfates et des sels d'acide sulfonique.

40. Sels d'addition d'acide selon la revendication 39, lesdits sels étant des sels d'halogénure d'hydrogène.

41. Sels d'addition d'acide selon la revendication 40, lesdits sels étant les sels de chlorure d'hydrogène, d'iodure d'hydrogène et de bromure d'hydrogène.

42. Sels d'addition d'acide selon la revendication 41, ledit sel étant le sel de chlorure d'hydrogène.
